(19) Europäisches Patentamt / European Patent Office / Office européen des brevets

(11) **EP 2 727 938 B1**

(12) **EUROPEAN PATENT SPECIFICATION**

(45) Date of publication and mention
of the grant of the patent:
**29.03.2017 Bulletin 2017/13**

(21) Application number: **11868642.7**

(22) Date of filing: **28.06.2011**

(51) Int Cl.:
**C07K 14/78** (2006.01)  **C08H 1/06** (2006.01)
**C08L 89/06** (2006.01)

(86) International application number:
**PCT/ES2011/070467**

(87) International publication number:
**WO 2013/001103 (03.01.2013 Gazette 2013/01)**

(54) **METHOD FOR OBTAINING COLLAGEN FROM ANIMAL SKIN**

VERFAHREN ZUR HERSTELLUNG VON KOLLAGEN AUS TIERHÄUTEN

PROCÉDÉ D'OBTENTION DE COLLAGÈNE À PARTIR DE LA PEAU D'UN ANIMAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**07.05.2014 Bulletin 2014/19**

(73) Proprietors:
• **Lanbide Heziketaren Lagunak, L.H.L**
**20570 Bergara-Gipuzkoa (ES)**
• **Belinchón Cantero, Pedro**
**20550 Aretxabaleta-Gipuzkoa (ES)**
• **Lasa Palacios, Mikel**
**20570 Bergara-Gipuzkoa (ES)**
• **Izco Urdanoz, Beatriz**
**20006 San Sebastián-Gipuzkoa (ES)**

(72) Inventors:
• **BELINCHÓN CANTERO, Pedro**
**E-20550 Aretxabaleta-Gipuzkoa (ES)**

• **LASA PALACIOS, Mikel**
**E-20570 Bergara-Gipuzkoa (ES)**
• **IZCO URDANOZ, Beatriz**
**E-20006 San Sebastián-Gipuzkoa (ES)**

(74) Representative: **Carlos Hernando, Borja**
**Garrigues IP, S.L.P.**
**Hermosilla, 3**
**28001 Madrid (ES)**

(56) References cited:
EP-A1- 1 270 672    EP-A1- 1 852 529
EP-A2- 0 256 663    WO-A1-2007/104322
WO-A1-2011/022794   DE-A1- 3 014 123
ES-A6- 2 017 564    GB-A- 1 361 540
GB-A- 2 079 797     US-A- 4 185 011
US-A1- 2011 020 864

EP 2 727 938 B1

**Description**

Technical Field of the Invention

The present invention relates to a process for obtaining a collagen material similar to rubber from animal organs, preferably from animal skin, as well as to the obtained product and the properties and applications thereof.

Background of the Invention

Collagen is a protein molecule that forms fibers, i.e., collagen fibers, found in all multicellular animals. They are secreted by the cells of the connective tissue such as fibroblasts, as well as by other types of cells. Collagen is the most abundant component in the skin and bones, making up 25% of the total protein mass in mammals. Today there is a lot of information about industrial processes relating to collagen manipulation.

The most conventional process is hide tanning. Skins of sacrificed animals are collected and hair is removed from these skins by swelling in basic reducing medium, leaving the skins ready for the subsequent operation of fleshing and splitting. In this operation the skin is split mechanically to separate the upper layer, with the pattern of the pore of the lower layer, which is the layer that was against the flesh. The first layer is tanned whereas the second layer is often disposed of. This second layer is called a split.

This waste is mainly used for obtaining adhesives, edible gelatins and meat casings. To obtain adhesives and gelatins, collagen is swelled in basic or acidic medium and gelatin, which is but a soluble, highly hydrolyzed, denatured (non-native), low molecular weight collagen, is gradually extracted by means of heat.

For meat casings, collagen is swelled in basic and acidic medium and then milled, obtaining an aqueous gel (5% solids). This gel can be extruded or calendered, as occurs in the paper industry with paper pulp (cellulose fiber gel). When processing collagen gel, collagen maintains its native structure because the denaturation temperature (denaturation being the collapse of the triple-helix structure) is not exceeded during processing. When collagen is denatured by boiling and is left to cool, maintaining it in an aqueous solution, it is converted into a very well known substance, i.e., gelatin.

There is also collagen foam which is obtained from skin that is first washed and then lyophilized. There is also collagen powder obtained by milling animal cartilage, which has healing value.

Therefore, it would be very useful to obtain a collagen material with an appearance similar to rubber but with advantageous industrial applications and properties.

There are several patent documents in the state of the art relating to obtaining collagen. Document US 4,185,011, for example, describes a process in which the starting material, animal skin, tendons and the like, is subjected to the following steps: digestion with a strong alkali such as calcium hydroxide until the amide nitrogen content is 0.2 to 0.4 moles/g, swelling the mass with an acid such as phosphoric acid, formic acid, sulfuric acid or hydrochloric acid to a pH of 0.5 to 1.5, mechanically separating the mass and deswelling by means of adding salts such as sodium chloride or organic solvents, dehydrating the collagen fibers to 20-40% of moisture based on the weight of the dry fibers. The obtained collagen fibers are used in the medical field.

Document US 4,404,033 describes a method for obtaining collagen fibers, the starting material and production process of which is similar to that described in the aforementioned document: said material is subjected to digestion with a strong alkali such as calcium hydroxide until the amide nitrogen content is 0.15 to 0.30 $\mu$moles/g and after a hydrochloric acid treatment and washing to a pH of 2 to 4, the swollen collagen is reduced in thickness under pressure, forming a two-dimensional layer. Finally, the deswelled fibers are treated with methylene diisocyanate in the presence of a non-ionic or cationic emulsifier and are processed in textile machines after dehydration. The obtained collagen fibers are used in the surgical field.

Document ES 457,823 relates to a method for obtaining an aqueous dispersion of collagen fibers having a diameter of 4-12 microns and a length of 2-25 mm, which comprises immersing the animal skin in a sodium hydroxide solution which also contains sodium sulfate, an amine and a monoheterocyclic compound at a temperature of 15 to 25°C for a period comprised between 5 hours and 10 days, washing with water, mechanically milling the material in an aqueous medium at pH 3.5-9.5 and temperature below 25°C for obtaining bundles of collagen fibers, which are then treated with a tanning agent for finally obtaining a dispersion of collagen fibers that are used in the medical field for obtaining tympanic membranes, kidney membrane, etc.

The object of document US 3,652,381 is a method for preparing a collagen material from tanned hide waste. This method consists of the following steps: immersing the starting material in a liquor containing water, sodium chloride and a strong acid, so that the material loses water; disintegrating the material until it swells to almost twice its initial weight; adding a sulfate or chloride solution and separating the obtained waste that is split into fibers.

Document EP 1 852 529 A1 relates to a method for extracting collagen fibers from products obtained from animal skins and to the machine for carrying out said process. The skin is washed in an ammonium sulfate solution and is cut into pieces that are immersed in a tank with a solution acidified with formic acid, being transformed into fibers in suspension after several hours. Water, sodium chloride and formic acid are then gradually added until reaching a pH of 2.5-3.5. Tanning solution is then added and a separation is produced between a solid phase and another liquid phase. The solid phase is treated with water and then rubbed with a roller until forming a bath made up completely of loose fibers.

Document ES 496,945 describes a method for preparing soluble collagen fibers from animal hide which comprises treating a raw hide with an aqueous solution of an alkaline metal hydroxide and an organic dehydrating agent to separate the hair and fat from same, providing a corium substantially free of hair and fat; stabilizing interfibrillar corium collagen bonds with an aqueous alkaline sulfate solution; neutralizing and dissolving the corium in an aqueous acid solution from which collagen fibers are obtained by precipitation and drying. These fibers are useful in edible foods or in solution and subsequently treated in a plurality of uses.

Document ES 2 340 606 T3 describes a thermoplastic composition comprising: a) 20% to 40% by weight of water, b) 10% to 20% by weight of plasticizer, and c) 40% to 65% by weight of dry collagen powder, i.e., the collagen that it comprises is a completely or partially denatured, fibril-forming dry collagen powder having a mean molecular weight of at least 500 kD, a solubility equal to or greater than 25% in water at 60°C and a mean particle size between 30 $\mu$m and 350 $\mu$m. Said document mentions the production of the thermoplastic composition, object of the invention, using dry collagen powder as an ingredient and that the process for obtaining said collagen powder is performed in three steps: soaking pieces of skin and passing them under pressure through plates with increasingly smaller holes, drying and denaturing at 60-80°C, and milling in a turbo rotor mill, leaving it as a powder having a diameter between 30 and 350 $\mu$m.

Document EP 0 256 663 refers to compositions and articles manufactured with such compositions characterized by comprising scrap treated skin and/or leather which is finely pulverized and mixed with synthetic resin. The aim of the invention being the provision of a skin/leather powder which does not agglomerate or flocculate, but maintains a granular state during the process of its manufacture, storage or when mixed with synthetic resin. Such skin and/or leather powder is obtained by the use of steam and pulverization techniques.

Document EP 1 270 672 refers to a process for recovering native collagen. This process does not contain a step of subjecting the skin product to a mechanical treatment step in mixing cylinders in the presence of a plasticizer and a neutralizing agent at a temperature of 50 - 70°C for controlled denaturation and molecular weight reduction of the collagen.

Document US 2011/020864 describes a method of extracting collagen from connective tissue by fist producing a collagen matrix and then extracting collagen from the said matrix by conventional methods to obtain a collagen containing solution. The method described herein contemplates the washing and swelling of the material, with a detergent, chelating agents, proteolytic enzymes, etc., but differs from conventional methods in at least the absence of rigorous physical or chemical treatments (e.g., grinding, homogenization, or harsh acidic/basic treatment).

Document GB 2 078 797 refers to a method of obtaining native isolated and purified collagen fibers. The process described herein does not include a step of mechanical grinding at a defined temperature while a controlled denaturation of collagen is performed.

Document WO 2011/022794 describes a process to extract the collagen protein in native form from bovine hide, which aims, amongst others, to avoid the undesired degradation of the collagen protein.

Document WO 2007/104322 discloses collagen powder as a precursor for the preparation of a homogeneous thermoplastic collagen based composition. This document describes obtaining the collagen powder and the thermoplastic collagen paste in two subsequent, distinct and separate processes.

Collagen is a fiber-forming protein molecule originating from a precursor protein called tropocollagen which is formed by three polypeptide chains known as "alpha strands". Each alpha strand is made up of a polypeptide formed by a repetition of about twenty amino acids among which the following three amino acids are the most abundant: proline, hydroxyproline and glycine. The triple helix remains bound by hydrogen bridges and some bonds between some amino acids (cross-linkings).

Type I to type XIII collagens have been described depending on where they are found and on their main function, however collagen in another state other than the native state, mainly amorphous collagen, having advantageous uses that are not present in conventionally processed collagen or common thermoplastics and rubbers, which is the object of the present invention, is unknown.

Object of the Invention

Therefore, the object of the present invention is the method for obtaining collagen obtained by a process in which rubber grinding technology is applied including a treatment of mechanically grinding the skin mainly at 50-70°C, and in which collagen which behaves like a thermoplastic or like a rubber depending on the plasticizer content is obtained from the animal skin. The physical properties of the collagen material thus obtained make it susceptible of being transformed by both thermoplastic and rubber transformation techniques, allowing a number of industrial applications of the material.

In some of the processes described above, when good mechanical performance of the end product is required, the attempt is made to keep the collagen in its native state with the highest long collagen fiber content so that products with a high molecular weight collagen content are later obtained when reconstructing same. The greater the final molecular weight the better the mechanical performance of the product.

On the other hand, to enable processing collagen in solid state, it must be partially or completely denatured, in a mainly amorphous, non-crystalline state.

In the process of the present invention, as described in section 7 "Processing the skin in mixing cylinders", the controlled denaturation begins with the addition of the whole skin that has been weakened but has a large amount of long fiber collagen. This means that the denatured collagen produced has a very high molecular weight when grinding is not excessive. In addition to improving mechanical properties, the high molecular weight allows adding plasticizers above levels in which there are more plasticizer than collagen (more liquid than solid), thus being able to obtain soft and stretchable gums.

Furthermore, the efficacy of batch mixers (mixing cylinders and Banbury) in terms of additive dispersion and homogenization is much greater than that of continuous mixers (extruders). Good ingredient dispersion and homogenization is vital for good properties of the end product obtained with those mixtures.

Therefore, based on the mentioned properties, collagen having advantageous technical novelties with respect to those described in the state of the art is obtained in the present invention.

Description of the Drawings

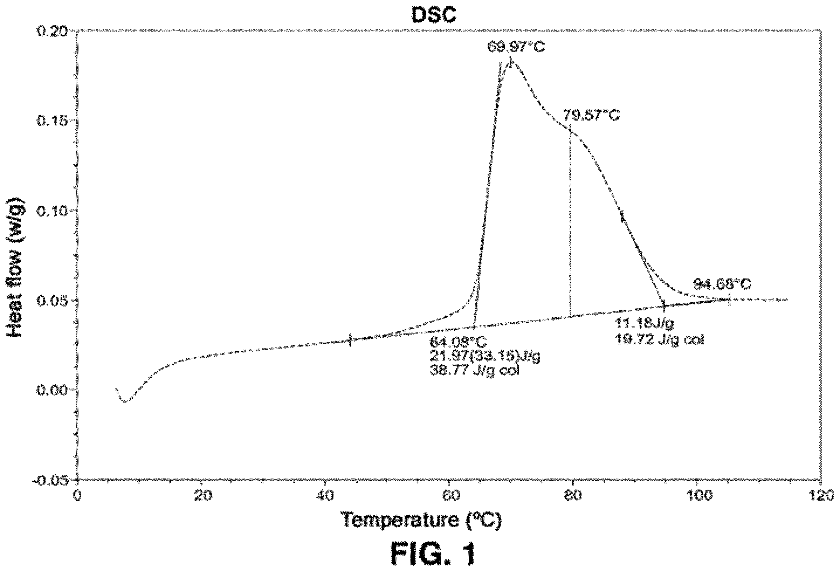

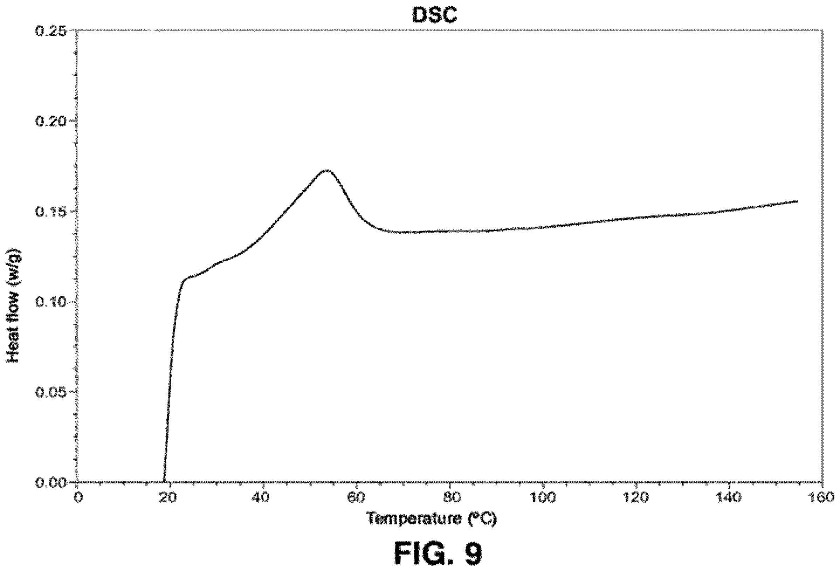

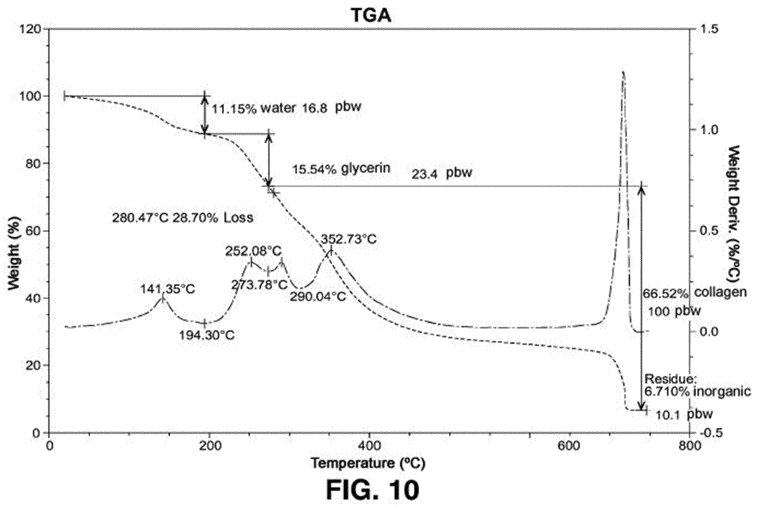

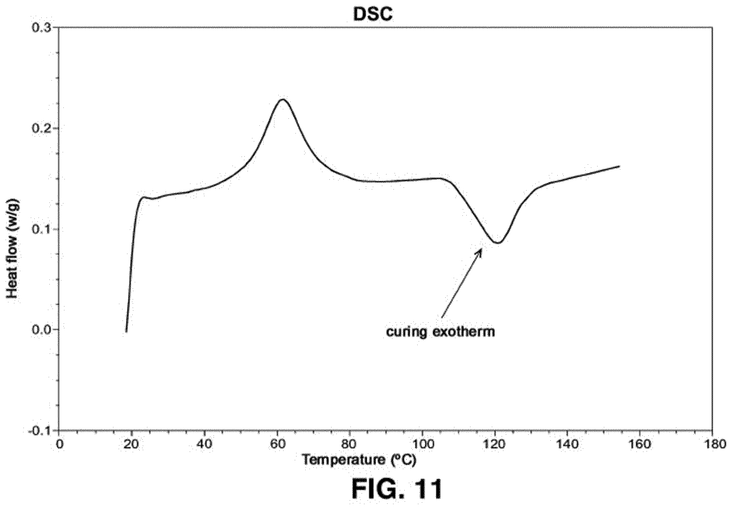

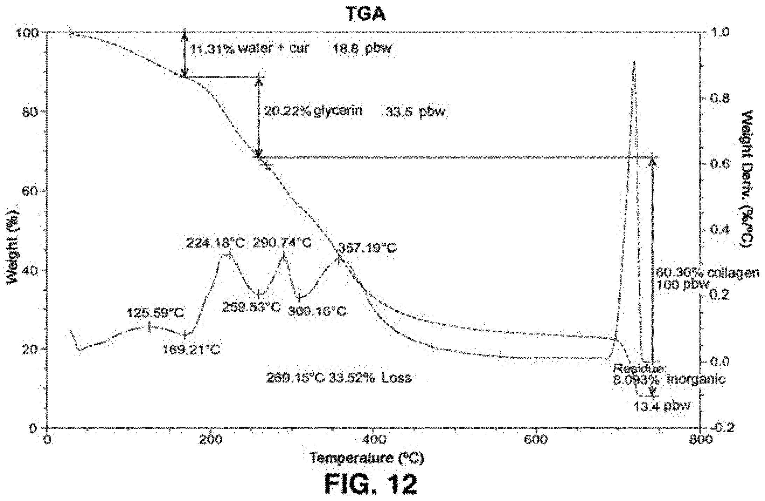

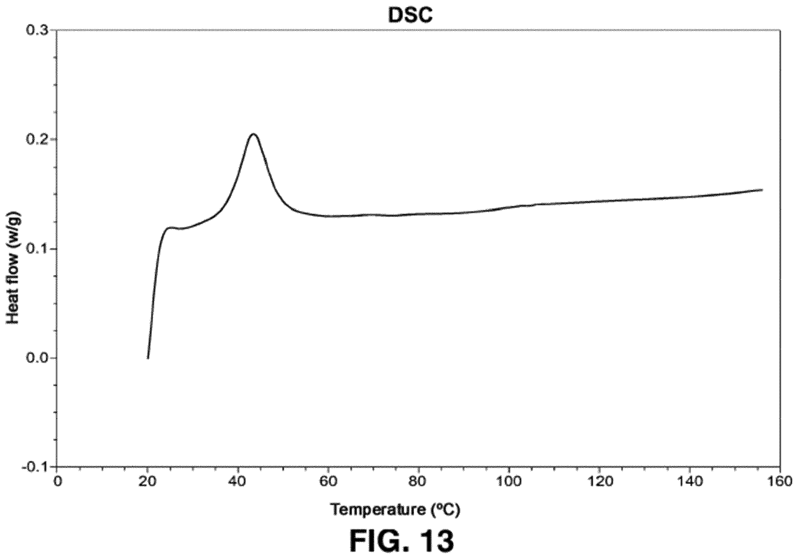

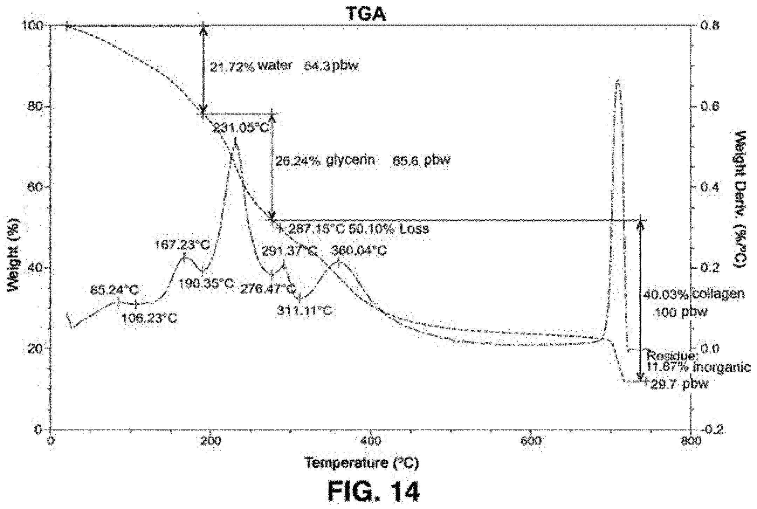

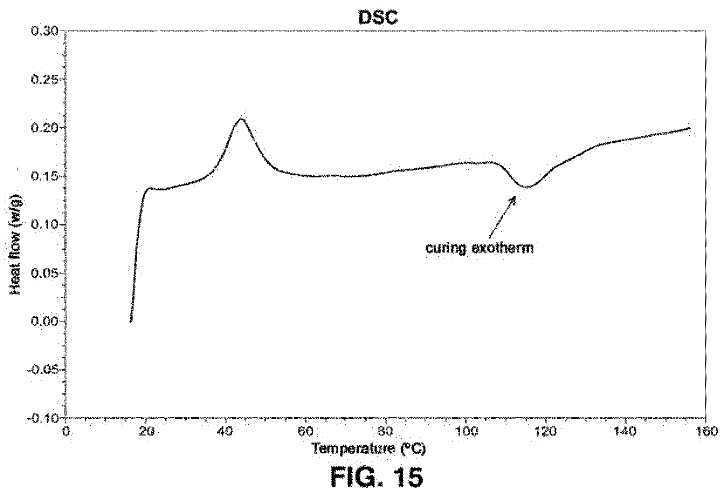

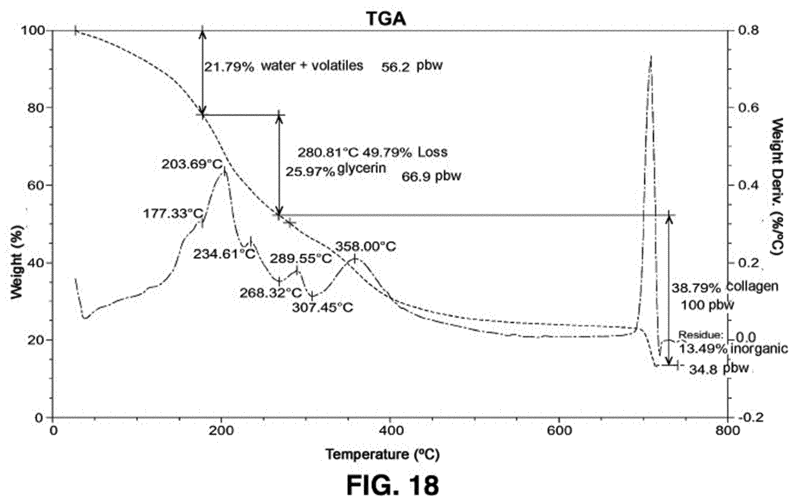

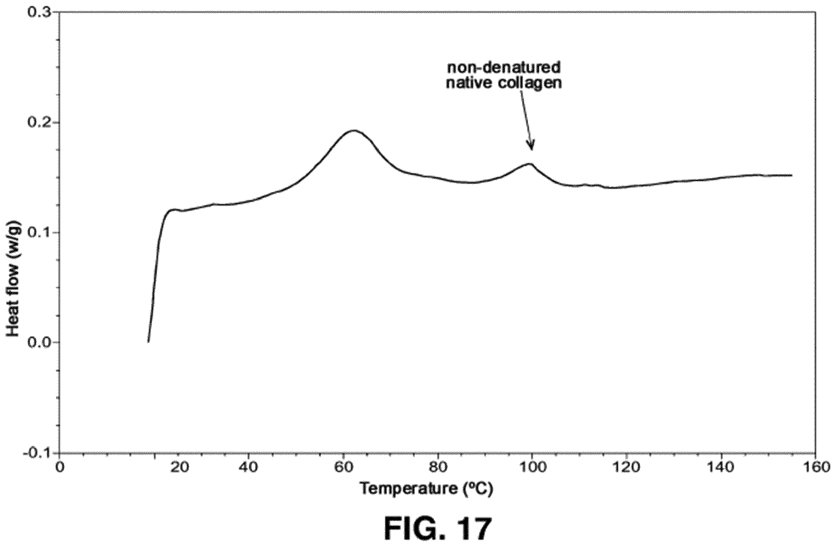

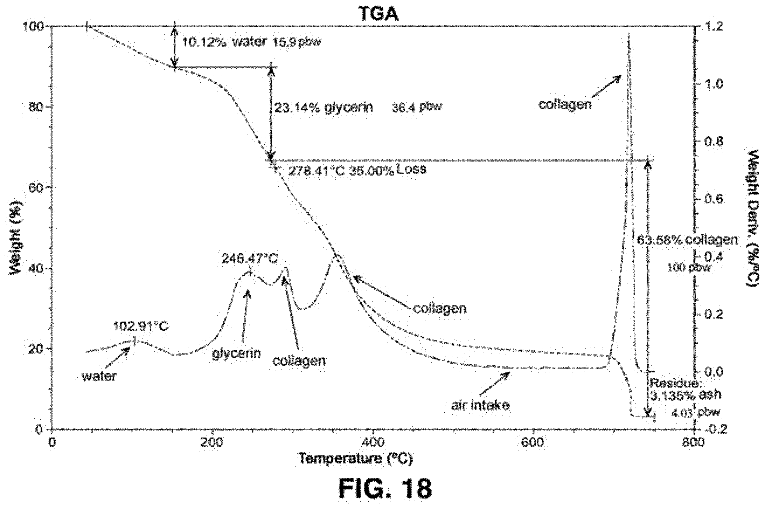

Figures 1 to 8 depict thermograms from DSC (differential scanning calorimetry) thermal analysis explaining the process and Figures 9 to 18 depict characterization by DSC and TGA (thermogravimetric analysis) thermal analysis of the different collagens obtained in the examples (DSC 2010 TA instruments and TGA 2050 TA Instruments).

Figure 1 shows collagen denaturation of soft areas.
Figure 2 shows collagen denaturation of hard areas.
Figure 3 shows the evolution of the hide with swelling of the soft area.
Figure 4 shows the evolution of the hide with swelling of the hard area.
Figure 5 shows thermograms of samples after wringing/drying without heat treatment.
Figure 6 shows the hide which is thermally treated and dried to the optimal level.
Figure 7 shows the hide which is thermally treated and dried excessively.
Figure 8 shows samples with two different grinding levels.

Figures 9 and 10 show Example 1: thermoplastic collagen.
Figures 11 and 12 show Example 2: curable thermoplastic collagen.
Figures 13 and 14 show Example 3: soluble elastomeric collagen.
Figures 15 and 16 show Example 4: curable elastomeric collagen (gum).
Figures 17 and 18 show Example 5: semi-ground collagen.

Detailed Description of the Invention

Before proceeding to the detailed description of the present invention, it must be clarified that the measurement (pbw) used in this description refers to parts by weight of ingredients or compounds calculated for every 100 parts of collagen (dry weight).

The present invention relates to a process for obtaining a thermoplastic collagen paste from isolated animal skins including:

1) Receiving and pre-treating the skins to obtain splits,
2) Washing the splits to remove toxic or unknown water soluble substances,
3) Swelling the skins to weaken the skin collagen,
4) optionally, subjecting the skins to heat treatment to shorten the swelling step,
5) Drying or wringing the skins to obtain a semi-dry treated skin having a water content between 35% - 65% and a hardness of 40 - 70 Shore A
6) optionally, shaving the skins for tearing off the weakest, innermost layer of the skins by means of a shaving machine and
7) Calculating the dry weight to calculate the amount of additives to be incorporated in the subsequent step, characterized in that the said process comprises a further subsequent step 8) comprising: subjecting the semi-dry treated skin of step 5) to a mechanical grinding treatment step in mixing cylinders in the presence of a plasticizer and a neutralizing agent at a temperature of 50 - 70°C for controlled denaturation and molecular weight reduction of the collagen present in the skin.

In particular, step 8 consists of the following steps:

8.1. Prior operations.
8.2. Obtaining the pre-paste.
8.3. Grinding the paste.

The obtained material is similar to rubber and behaves like a thermoplastic or like a rubber depending on the plasticizer content.

Thus, the present invention also relates to a thermoplastic collagen paste suitable for press molding, extrusion molding and injection molding obtainable by the process of the present invention, characterized in that it is in the form of a soft and stretchable gum, is biodegradable and recyclable and comprises denatured collagen, a plasticizer agent a neutralizing agent and optionally, additional additives selected from the group consisting of curing agents, cross-linking agents, accelerators and/or activators, internal or external lubricants, foaming agents and reinforcing fillers.

For an amount of 100 pbw of glycerin or ethylene glycol in 100 pbw of collagen, the material behaves like a rubber (gum), whereas for 20 pbw of glycerin or ethylene glycol and 100 pbw of collagen the behaviour is similar to that of a thermoplastic. Like PVC, it can be rigid (window frames) or soft (beach balls) by simply changing the plasticizer content between 0 and 100 pbw, referring to 100 pbw of PVC.

This behavior of the collagen of the present invention means that it can be transformed with thermoplastic and rubber transformation techniques. In other words, by increasing the temperature the collagen softens, is molded and the new shape consolidates upon cooling, or a system that produces cross-linkings by means of heat is added, collagen gum being obtained.

The innovative aspect of the process is based on applying rubber grinding technology to collagen. In other words, step 8 (processing the skin in mixing cylinders) contains the novel key step. Evidently, to be able to process the skins, they must be prepared by means of processes already described in the literature, but with original variants.

This processing in mixing rollers means that many different additives such as plasticizers (water, glycerin, lubricating oils, vegetable oils, etc.) and reinforcing fillers (calcium sulfate, calcium carbonate, precipitated silica, cellulose fibers, native collagen fibers, other polymers, etc.), can be added, even in large amounts.

A cross-linking or curing system can also be added: a mixture of products containing a cross-linking agent, an accelerator and/or an activator. The cross-linking agent can be ionic (zinc peroxide, $ZnO_2$ or covalent (tetramethylthiuram disulfide, TMTD), and the activators can be divalent metal oxides (zinc oxide, ZnO, magnesium oxide, MgO). This system is relatively inert to the processing temperature in the rollers, but it decomposes between 100-150°C by introducing cross-linkings that keep the polypeptide chains bound either covalently (stable) or ionically (removable with water).

Other possible additives can be internal or external lubricants (organic acids, acetic acid, stearic acid, etc.), foaming agents (ethanol), etc.

All this means that the end collagen can have very different properties and be intended for a number of uses. For example, if active ingredients are incorporated, they can be used as an active ingredient carrier in the pharmaceutical and cosmetic industry (healing agents, analgesics and others). In the agricultural industry, they can be used with fertilizers, fungicides and others. In the food industry, other proteins and biopolymers (albumin, cellulose, starch, etc.) as well as dyes, flavorings, preservatives, etc.

Obtaining the collagen

1. Receiving the skins

The skins are received after removing hair with a reducing treatment at basic pH (pH 12-13 for one day) (liming) and fleshy remains (fleshing) at the point of origin, and they are then subjected to the splitting operation. In other words, the inner part of the skin or split, where the most weakly cross-linked collagen is located, is received. This method is suitable for any skin, and the part of the animal where the skins come from is not a determining factor: belly, butt or neck. The softest areas will need less subsequent swelling time, and/or less or almost no heat treatment. If the part of the hide received is very heterogeneous (the belly), it can be separated upon reception or after swelling.

DSC thermograms prepared for characterizing the process for obtaining collagen can be seen in the drawings attached to the present specification.

In the differential scanning calorimetry, the sample to be analyzed (10 mg) is subjected to a heating program (for example at 5°C/min) and the calorimeter records the amount of heat given off or absorbed by the sample and the temperature at which it occurs. The occurring physical and chemical phenomena in which there is heat exchange can thus be studied.

Collagen denaturation is an endothermic process (there is a rather wide peak above the baseline), whereas curing is an exothermic process (peak below the baseline).

The following can be determined from the endothermic denaturation line:

- Denaturation onset and offset temperatures: take off from and return to the baseline.
- Rapid onset temperature (extrapolated onset) and the most representative temperature of the process (maximum heat exchange rate), or peak temperature.
- Heat involved or denaturation enthalpy.
- Shape and width of the endothermic line.

Figure 1 shows a thermogram of collagen denaturation in the soft areas of the skin, having a hardness of less than 20 Shore A (Shore A Baxlo durometer), performed in the calorimeter. It is seen therein that starting from 50°C, the thermogram deviates from the baseline (denaturation starts) and returns to the baseline at about 100°C (denaturation ends). In this case, the analyzed sample is from a recently received skin: pH 12-13 and levels of water between 70-80%.

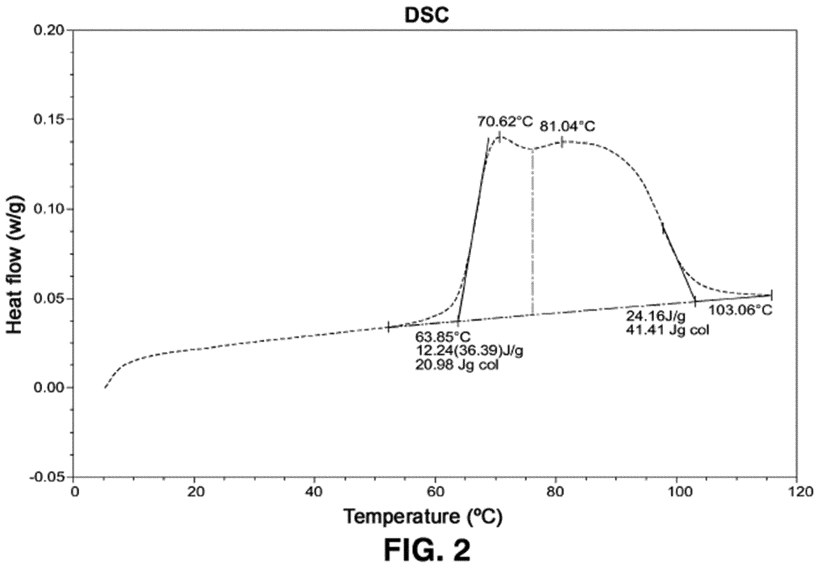

The test conditions in Figure 2 are the same but for a hard area of the skin, having a hardness greater than 25 Shore A. The following differences are observed:

- The denaturation process onset and offset temperatures increase.
- The second peak is wider and is about twice the width of the first peak. It was half in the preceding case.
- The denaturation enthalpy is slightly higher.

All these facts suggest that collagen in the hard areas is richer in natural cross-linkings, has a higher molecular weight and a higher content of native structure than the collagen in the soft areas.

### 2.- Washing the splits

The purpose is to remove any toxic or unknown water soluble substance and to prepare the splits for subsequent treatment. The degree of swelling of the collagen, i.e., the high level of intramolecular water, 70-80%, and low hardness, less than 30 Shore A, must be maintained. To that end, they are washed in a high pH aqueous solution similar to that used in the subsequent step. While sodium hydroxide is used in this washing step, a 2 g/l calcium hydroxide solution is used in the subsequent washing step.

### Method

1. The skins are introduced in the tanning drum, for example, a Demar D-120 drum.
2. The previously calculated sodium hydroxide is added such that the final concentration in the washing water is 2 g/l.
3. Water is added until it completely covers the hides, between 1.5 and 2 times the weight of the hides, and the pH is verified to be between 12.6-12.9.
4. Washing efficacy is higher if the water is hot (not more than 40°C).
5. The drum is rotated at about 10 rpm for one or two hours.
6. Skins are wrung for 15 minutes.
7. Steps 2, 3, 4 and 5 are repeated two or three times.
8. Before the last wring, the pH is checked to assure that it has not dropped below 12.6.
9. After the last wring, the skins are prepared for swelling.

### 3.- Swelling the skins

The purpose of swelling is to weaken the skin collagen as much as possible without destroying its native structure and damaging the skin as little as possible.

The higher the treatment temperature the shorter the treatment time. The treatment time depends on several factors. The time is longer the thicker and harder the skin is. Skins 3 mm thick and having a hardness of less than 20 Shore A (soft area of the bellies) will be ready for the next step in three days. Skins more than 1 cm thick from the hard area of the belly (greater than 25 Shore A) will need 6 days.

The end of treatment is established after an inspection: the skins can be easily cut with scissors and some areas can also be ripped using hands (although with difficulty). The hardness must be less than 20 Shore A after treatment.

The denaturation temperature in the collagen drops: the low rapid onset temperature (extrapolated onset temperature) drops from 60-65°C to 50-55°C, and the shoulder or the double peak after the main peak which appears in a DSC thermogram is smaller than the preceding one.

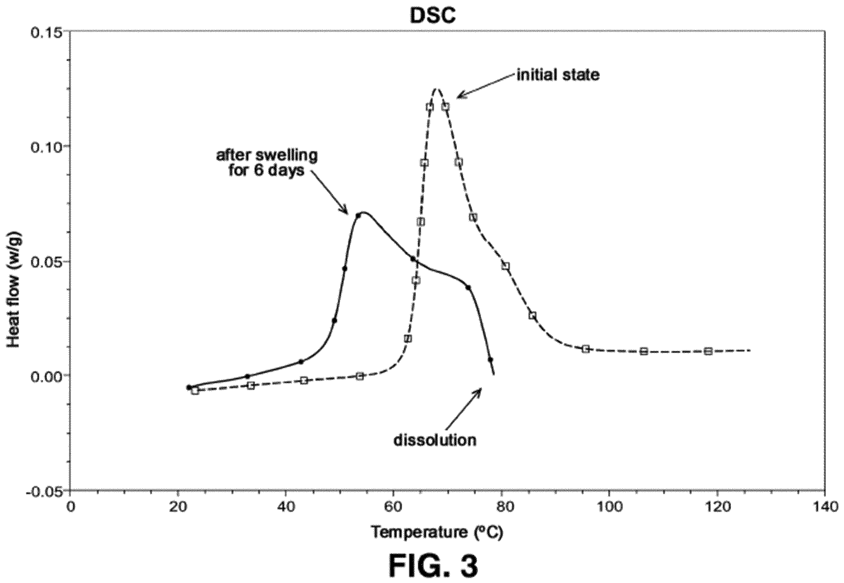

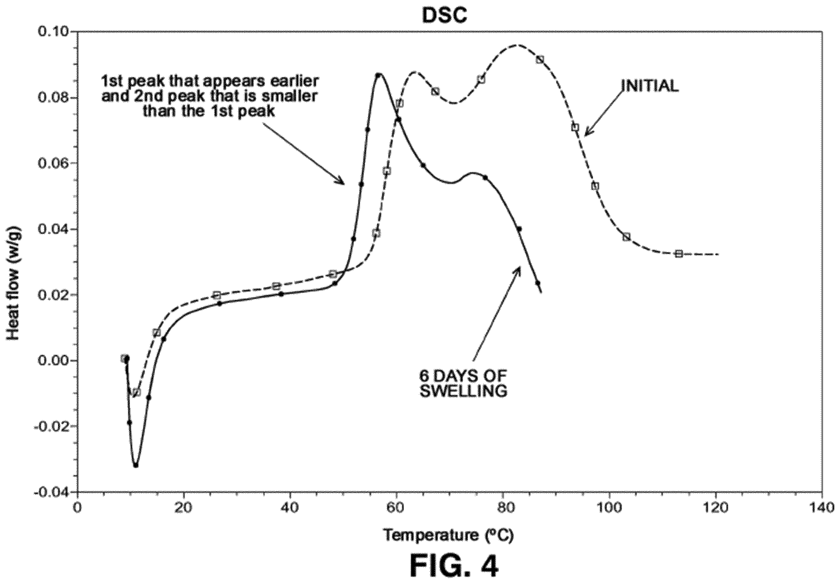

Figures 3 and 4 show thermograms with the evolution of the native collagen throughout the swelling period:

- The denaturation temperature in the collagen from both areas drops: from about 65°C to about 50°C for the soft area and from 65°C to 60°C for the hard area. Enthalpy drops in both cases from 55-65 J/g to 40-50 J/g.
- In the swollen state that occurs after treatment and at that high pH (12.6-12.9), it is observed that hydrolysis and dissolution occur after denaturation during DSC scanning.

Dissolution is used for preparing soluble gelatins used in food and for adhesives. In this method said dissolution in water is avoided. For this reason, the skins are subjected to subsequent wringing or drying and to neutralization in the rollers while the controlled denaturation in step 8 is being performed.

Not moving the skins around excessively, not allowing the temperature to rise above 30°C, and not prolonging treatment for more than 8-10 days are enough to prevent said denaturation and dissolution from occurring in the swelling treatment. The standard conditions of the present method are: from 3 to 6 days at 20°C, and a drum rotational speed of 4-6 rpm with intermittence of stopping for one hour and rotating for one minute (so that the calcium hydroxide does not decant).

Method:

1. Calcium hydroxide is added on the wrung skins in the tanning drum used in the preceding step. The amount of Ca(OH)$_2$ is the amount calculated previously for obtaining a 2 g/l final concentration of the aqueous swelling solution.
2. Running water is added until it completely covers the hides, between 1.2 and 1.5 times the weight of the hides.
3. The drum is left to rotate slowly (4-6 rpm) at intermittent periods: stopping for one hour, rotating for one minute, for 3 to 6 days.

4.- Heat treatment (optional)

The aim is to subject the hides to heat treatment between 45 and 55°C at the same pH as the preceding step (12.6-12.9) in order to shorten the swelling time, facilitate subsequent processing in the rollers and shorten the grinding time. The time and temperature of this heat treatment depend on how long the skins were swelled in the preceding step, as well as on their thickness. The longer the swelling the lower the temperature, the thicker the skins the higher the temperature. If the skin is from a hard area and is thick (1 cm) with 6 days of swelling, it will be necessary to reach almost 55°C. For soft skins of less than 5 mm thick and after 3-4 days of swelling, the heat treatment will be milder and will not need to exceed 50°C.

Mild partial collagen denaturation occurs in this process: the skins become slightly twisted, remain intact but can be easily ripped with hands. They must still be soft and the solution becomes slightly sticky.

This treatment is not completely necessary but the gelatin formed and retained inside the skin favors processing in the mixing rollers, so this step is included in the method of the present invention.

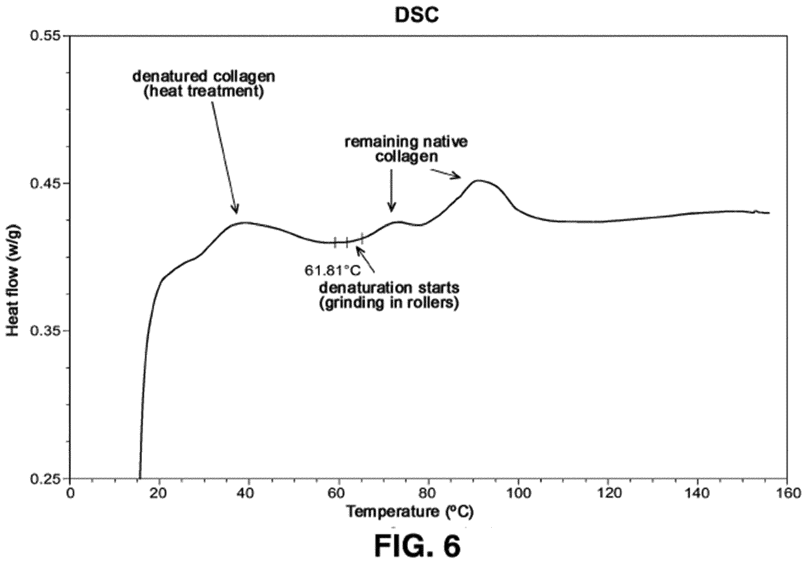

DSC (Figure 6) shows a melting peak of the gelatin formed in the heat treatment before the main peak, at about 35-40°C.

Method

1. Most of the solution is removed from the tanning drum, keeping the skin at its maximum degree of swelling. As long as they are not wrung excessively, the collagen incorporates the maximum amount of water possible, greater than 80%, for that pH of 12.6-12.9 after a correct swelling treatment during step 3, after which the skins are inspected.
2. The grating is closed and the temperature is raised to 45-55°C, rotating the drum very gently (2-3 rpm) until the skins reach those temperatures (length of time in the drum varies according to drum size and amount of skins). The heater is switched off, the grating is opened and the skins are left to wring for half an hour at the same rotational speed.

### 5.- Drying or wringing

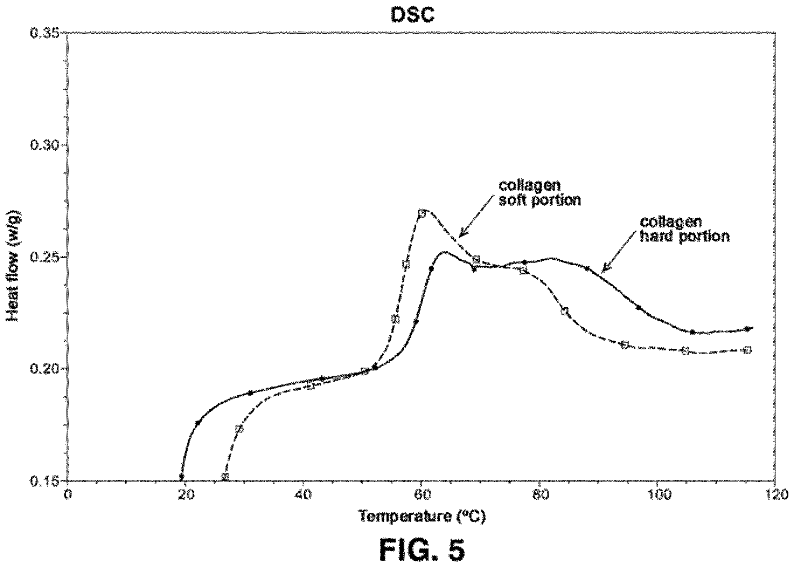

If the heat treatment has been omitted, the collagen denaturation onset temperature with those levels of water must not exceed 60°C (DSC, 5°C/min) after this step. Figure 5 shows the thermograms of two dry samples (without heat treatment) in the optimal hardness level, ready for processing in the rollers of step 8.

The aim is to remove water from the skins, causing the skins to have a level of water of between 35% and 65% corresponding to a hardness of 40 to 70 Shore A, regardless of whether heat treatment has been performed. If the level is greater than 65% the grinding will last too long, and if it is less than 35% they cannot be suitably processed.

Figure 6 shows the thermogram of a sample from a hide after heat treatment and drying. The thermogram shows a peak before the main peak. It is the melting of the collagen already denatured during heat treatment. That part of the material is what allows the hide to stick and hold onto the rollers quickly and facilitates denaturation and grinding by friction and heat during subsequent processing in the rollers. It has a hardness of 40-45 Shore A.

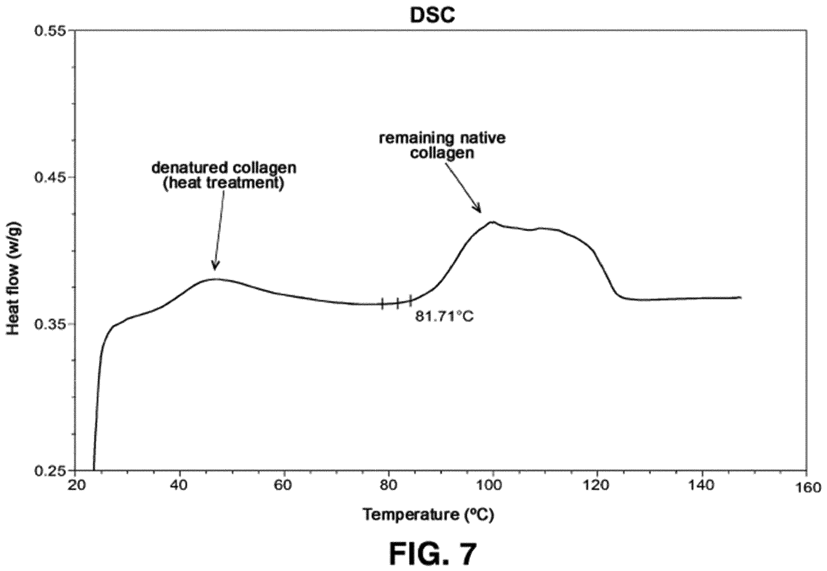

In Figure 7 the hide has been dried excessively (25% water): it will not readily adhere to the rollers. The rollers would have to be heated up excessively, above 80°C, which would lead to quick water evaporation that would dry the hide further, stabilizing the collagen even more (the denaturation peak would shift to higher temperatures). Hence, it is impossible to process same. It has a hardness greater than 70 Shore A.

For drying, the skins are removed from the drum and stretched as much as possible in order to prevent creases and taut areas that dry quickly. Depending on the temperature and on air renewal in the room, drying can last from 12 hours to 5 days. For example, if they have 80% water and the hide is more than 1 cm thick, they would take between 3 and 5 days to dry. For that same water content and thicknesses of less than 3 mm, they are ready for the subsequent step in just one day.

There is machinery in the traditional leather industry, the wringer, in which the skins are pressed by means of two rollers covered with an absorbent fabric. They are wrung to a larger or smaller extent depending on the pressure. This last alternative consumes more operating time, but the total cycle time is shortened and drying is more homogenous.

### 6.- Shaving step (optional)

Before final processing, i.e., when the skins have a hardness between 40 and 60 Shore A, they can be subjected to an additional step called shaving, which is carried out in a shaving machine, such as a Flamar Spa Ran 1S shaving machine, for example.

This step consists of tearing off the weakest, innermost layer of the skins by means of a shaving machine (such as those used in the leather industry) in the form of shavings for subsequent processing. These shavings are processed and the remaining layer is disposed of.

It has the advantage of reducing the processing time in the mixing rollers, the most complicated step which takes up more of the operator's time. It has the drawback of material waste.

### 7.- Calculating the dry weight

It is necessary to calculate the amount of dry weight of semi-dry skins in order to calculate the amounts of additives to be incorporated in the subsequent phase such that said additives are present at the suitable levels required in the end product. This step of the process is carried out with a Selecta Digitronic forced-air oven and a Gram Precision ST-71 precision balance.

It is measured gravimetrically, releasing water and assuming that what remains in the residue is almost all collagen with some inorganic salts.

All weight measurements must have 3 or 4 representative figures so that the results have a relative error of less than 1%.

### Method

1. 5 to 10 watch glasses are weighed and Wg is recorded.
2. A piece of sample is added in each watch glass and it is weighed again, Wgs
3. It is introduced in an oven for 1 hour at 150°C.
4. They are taken out and put in the dryer until recovering room temperature.
5. They are weighed again, Wgd.
6. An Excel table is filled out and it is calculated by means of formula:

$$\% \text{ of water} = ((Wgs-Wgd)/Wgs) \times 100$$

The mean of 5 to 10 measurements is also calculated.

| Wg | Wgs | Ws(Wgs-Wg) | Wgd | Wd(Wgd-Wg) | % of solids (Wd/Ws)x100 | % of water ((Wgs-Wgd)/Wgs)x100 |
|----|-----|------------|-----|------------|-------------------------|--------------------------------|

<u>8.- Processing the skin in mixing cylinders</u>

The objective of the process is to take collagen from its native triple-helix protein structure to a different linear structure so that it can suitably accept significant amounts of various additives and so that it is capable of flowing under pressure and heat (capable of forming fibers in subsequent processes, such as in nylon thread extrusion). In other words, the aim is to produce controlled denaturation preventing the collagen from being excessively hydrolyzed and preventing it from reorganizing into other more heat-stable structures. Hereinafter the combination of the denaturation and molecular weight reduction processes which correspond with the present step of the method will be called grinding. This step of the process is carried out in mixing cylinders, such as Meccaniche Moderne Gobi 1500 mixing cylinders.
To that end, the following steps are carried out:

8.1 Prior operations

Plasticizer is the additive that must always be present in all the formulations of this collagen. Therefore, the skin from the preceding drying step is weighed and the relevant calculations are made to determine the amount of plasticizer (glycerin, ethylene glycol, lubricating oils, DOP, etc.) that must be added to achieve the desired levels in the end product.

<u>Example:</u>

For a soft final paste for 100/100 (collagen/plasticizer) gums, the preferred plasticizers in this case being glycerin, ethylene glycol and/or lubricating oil

100 g of skin $\rightarrow$ 33 g of collagen $\rightarrow$ 33 g of plasticizer

For a 100/30 (collagen/plasticizer) rigid thermoplastic final paste, the preferred plasticizers in this case being glycerin, ethylene glycol and/or lubricating oil

100 g of skin $\rightarrow$ 33 g of collagen $\rightarrow$ 9.9 g of plasticizer

The plasticizer can be added directly or diluted with water when they are miscible, such as for example: a 50% by weight solution of glycerin in water, or in emulsion when they are not miscible, such as for example: a 50% by weight emulsion of water and lubricating oil.
Plasticizer diluted with water is added because it is incorporated better that way. If any other solid was to be added (curing agent, such as ZnO and TMTD, or reinforcing filler such as $CaSO_4$), a prior mixture, preferably a semi-solid mixture, is made because the incorporation is much faster in this manner.
The second necessary additive is the neutralizing agent: ammonium chloride or sulfate.
The amount of ammonium sulfate, $(NH_4)_2SO_4$ or $(NH_4)Cl$ necessary for neutralizing a specific skin sample swelled in the presence of phenolphthalein is measured in an earlier volumetric analysis. For example:

100 g of skin need 5 ml of 2 N aqueous solution of $(NH_4)_2SO_4$ or $(NH_4)Cl$, which can also be added as a solid but the dispersion is worse.

The amounts of other additives such as curing agents, reinforcing fillers, process aids must also be calculated, depending on the final characteristics to be given to the end product. If 5 to 10 pbw of lubricating oil (sulfated vegetable oil, for example) are added, the collagen will be more resistant to abrasion and stretchable. If calcium carbonate or sulfate are added in the same amounts, it will give rise to a tougher material. If 3-6 pbw of curing system are added along with up to 100 pbw of plasticizer, it will give rise to softer gums.
Every time an ingredient is added, said ingredient must be incorporated, dispersed and homogenized as well as possible to achieve the optimal final properties. There are two important operations and performing them repeatedly is of interest:

- Cutting: the band, i.e., the somewhat continuous material covering the front or rear cylinder, is horizontally cut from one side up to two third the length and placed on the other third and vice versa.
- End pass: the band is removed completely, rolled up and introduced between the two rollers again.

The starting hide must go through the steps described above and the mean hardness of the hide must be between 40-70 Shore A. Lower hardnesses prolong the grinding process and greater hardnesses excessively increase the denaturation temperature making the processing thereof in the rollers difficult. However, particularly and for effective grinding it is important for the hide to be homogenous, not having differences in hardness that are greater than 10-15 Shore A. Otherwise, a prior classification is made. The rollers must first be heated to 50-60°C depending on the hardness of the skin to be processed.

Optional prior operation

The addition of plasticizer in the mixing rollers as described in paragraph 8.2 and in the examples is a relatively complicated operation. It is the additive that is the most difficult to be incorporated. To that end, the entire additive amount or part of it (particularly for large amounts of plasticizer in the end collagen) can be incorporated in an optional step.

After the wringing or drying step 6, the skins are introduced in the drum and are again subjected to swelling in a 0.1 N solution or emulsion of sodium hydroxide (NaOH) in plasticizer and water.

Due to the initial swelling step (step 3), the collagen is somewhat hydrolyzed. The foregoing and the high pH (12-13) of the swelling solution cause the hide to absorb said solution with relative ease.

Example:

1. The semi-dry skins (hardness of 20-80 Shore A) are introduced in the tanning drum.

2. 1.2 to 1.5 times by weight of a pH 12.6 solution of glycerin in water is added. This solution is obtained as follows (for 5 liters):

a) About 2 liters of water and 20 g of caustic soda are poured into a 5 liter container and stirred well to complete dissolution.

b) The necessary amount of glycerin (previously calculated in this section) is added and stirred well.

c) The solution is topped up to the limit of the container.

d) The resulting pH is checked to be between 12 and 13.

3. The drum is tumbled at low speed (3-4 rpm) for at least 3 hours or until it is observed that there is no more liquid in the drum (the skin has absorbed all the liquid).

4. The drum is made to rotate for one minute and rest for 15 minutes for the rest of the day (so that the mixture becomes homogenous).

5. The skins are taken out and stretched out for subsequent drying which will provide said skins with the optimal hardness required for the next step.

8.2 Obtaining pre-paste

1. The rollers are opened completely, maintaining the pitch (distance between rollers) similar to the width of the skins. The skins are passed through a few times, 5 to 10 times, without closing the rollers, until it is observed that the skins start to stick to the rollers (5 minutes). It is very important that the skins are not wrung. The optimal temperature of this step is between 50-60°C.

2. The rollers close little by little as the skins lose water and the band being formed becomes thinner. In this step it is important to have a little shear. The material preferably passes through little by little without being retained or crumbling. The longer the time the material is kept intact, the better it is. The hide becomes softer in 5 or 10 minutes.

3. The rollers close gradually increasing the shear (from 5 to 10 minutes).

4. The plasticizer is added little by little when the band and the roll (material accumulating between and on the two rollers that rotates continuously due to the different speed of each of the two rollers) are formed. It is important to perform this operation slowly so that the paste does not crumble. The neutralizing solution is then added, waiting for the material to form a web (from 5 to 10 minutes). Throughout this entire time, the temperature of the material must not exceed 70°C.

It is important to track the temperature control throughout the entire cycle (measured at the center of the band). If it exceeds 70°C, it is cooled down by adding water or ice. If the temperature drops below 50°C, the process becomes longer.

After the cycle of 20-35 minutes the paste is ready to be ground.

8.3 Grinding the paste

Two options are contemplated:

1. Grinding the paste continuously to minimum viscosity which is detected because it starts to shine. It is necessary to reach 80°C (if the paste does not reach this minimum viscosity, it neither shines nor flows at 80-90°C in a compression press). To that end, it is kept for a longer time in the rollers, cooling it once in a while with water or ice. In this manner, it will be ready to be processed by extrusion or injection. Furthermore, the rollers will be hot enough so as to start another cycle with other skins. The paste must be removed with the rollers in rotation and completely opened so as to not pinch or orient it.

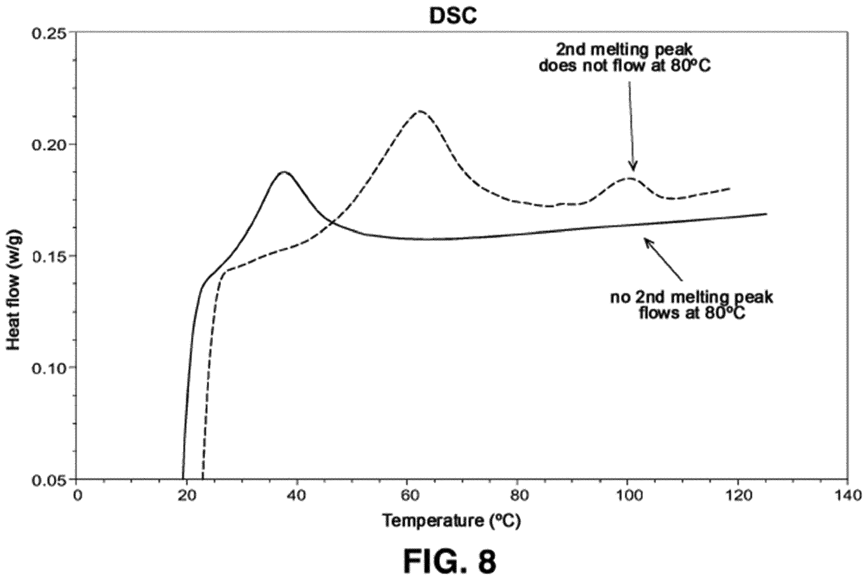

The continuous line in Figure 8 represents the thermogram of a sample sufficiently ground for subsequent processing by extrusion, compression or injection. It shows the presence of a single peak corresponding with the melting of the collagen that has recrystallized upon cooling after removing it from the rollers. For a specific level of water and additives, the position and the size of the peak are indirect measures of the degree of grinding achieved and of the final molecular weight. For example, for a liquid (water plus plasticizer) content of 50%, the peak is between 40-50°C and enthalpy is between 10-20 J/g, depending on the degree of grinding.

Collagen is relatively stable in this state (it tends to continuously recrystallize over time), does not decay and can be stored for months or even years.

2.- Removing the paste with little grinding at 65-75°C (this is the second option if the paste is not to be ground completely) (pre-paste), for working with it subsequently in cold conditions, finally adjusting the levels of plasticizer, water and other additives. (If grinding is performed in a single step, option 1, the material must have the same level of all the additives than if it is performed in two steps, option 2. In this case it is better to add the curing agent and the remaining plasticizer and additives in the second step). The solid content can thus be reduced to levels of less than 50%, which will allow obtaining articles made of gum and foam articles. Like in option 1, collagen is stable in this state, does not decay and can be stored.

The dotted line in Figure 8 represents the thermogram of a paste that is not ground completely. It contains crystalline domains corresponding to native (non-denatured) collagen. This crystalline area prevents subsequent processing by injection or extrusion. The degree of denaturation progression is given by the ratio between the areas of the two peaks: the greater the second peak and the smaller the first peak, the less it has been ground.

Summary of the most important considerations

1. The semi-dry treated skin must have a hardness between 40 and 70 Shore A before the process. Below 40 Shore A, the cycle becomes much longer because the material has a lot of water and the temperature drops excessively. Above 70 Shore A, water must be replenished at the beginning so that the temperature drops below 60°C.

2. A good temperature range for collagen denaturation while grinding is between 50°C and 60°C. Molecular weight reduction is more intense when the temperature of the system starts increasing due to internal friction.

3. Neutralization is suitably performed before reaching 70°C so that there is enough water for the reaction.

4. As a general rule, the addition of solids during denaturation and grinding aid the process but the addition of liquids delays the process. Liquids reduce system viscosity: internal friction is reduced, grinding is less effective and the temperature increase is delayed.

5. Due to that described in the preceding paragraph, a good addition of plasticizer is very important. When the hides are homogenous, i.e., have the same hardness everywhere, plasticizer can virtually be added from the start, when the collagen fibers are still visible (denaturation has virtually not begun) as described in section 8.2., alternating same with the addition of reinforcing filler if any. The incorporation thereof is much faster (less than 5 minutes) in this manner and the cycle time is shortened by 10-15 minutes.

When the hides are heterogeneous (areas with different hardness), early addition of plasticizer causes separation into two phases: the softer areas absorb the plasticizer faster and in a larger amount whereas the hard areas remain intact, the denaturation and grinding thereof being very difficult. Lumps of native collagen appear in the end collagen.

6. For this reason if the hides are not homogenous, it is essential to delay as much as possible the addition of plasticizer, when the band and the roll start to become shiny.

7. The denaturation and molecular weight reduction processes are simultaneous, although denaturation predominates over molecular weight reduction at first. High temperatures favor denaturation, whereas mechanical shearing favors molecular weight reduction. By varying the temperature and opening and closing the rollers, the progress of either process can be controlled. In other words, completely or partially denatured, high molecular weight pastes as well as completely denatured, low molecular weight pastes can be obtained.

8. In the last grinding phase water replenishment must be combined with temperature increases. Effective grinding involves the destruction of the last crystalline collagen clusters. This destruction is as difficult at a high temperature with little water as it is at a low temperature with a lot of water because collagen tends to recrystallize when the temperature is high and there is little water.

9. Addition of 3 to 5 pbw of $CaSO_4$ or ZnO make the grinding more effective and quicker because they prevent collagen from reorganizing during denaturation, in addition to stabilizing collagen in the final part. Addition of 1-2 pbw of aluminum stearate or stearic acid helps to remove the paste from the rollers (it peels off more easily).

10. If denaturation is complete, the final paste must be off-white, shiny, smooth and homogenous. It must not have a second melting peak above 45-65°C (see Figure 8) and must flow in a compression press at 80-90°C.

11. It must be taken out before reaching 85-90°C. Above said temperature, the paste loses structural water and hardens excessively. If it has a curing system, it can be cured and harden. If said temperature is reached and it is not ground completely, the temperature is reduced with water or ice and grinding continues until it becomes shiny and off-white.

12. If the cycle is performed continuously and completely, band formation and neutralization are achieved in 15-20 minutes, whereas grinding can take another 15-30 minutes. If there is no possibility of cooling the rollers, it is better to form the band and neutralize, grinding in cold conditions and adding the curing system (if any) the next day.

13. The times provided are estimations and it largely depends on the quality of the mixing rollers: whether or not the differential speed of the cylinders can be controlled and on whether or not there is a possibility of cooling same. This can make the total cycle time (obtaining the pre-paste plus grinding) be less than 15 minutes or more than half an hour.

14. When the grinding is not complete, crystalline structures with melting temperatures above 70°C remain. The collagen cannot be subsequently molded below 100°C. Then there are two options:

a) Increasing the molding temperature above 120°C (this temperature depends on the final degree of grinding) to enable processing it in a compression press. This can be done depending on the curing system (if the curing reaction temperature is higher than 150°C) and if there is a possibility of discharging the evaporated water (while processing by extrusion, for example).

b) If the preceding option is not possible, more plasticizer is added until the level of liquids is similar to the level of solids in the collagen, so that it can flow at temperatures below 100°C. Subsequent molding of these pastes (in both a and b) produces tougher and more rigid products.

15. Highly ground pastes are suitable for press molding, extrusion molding and injection molding. Moderately ground pastes are only suitable for compression molding unless they are brought to a level of liquids that is at the very least similar to the level of solids. A highly ground paste is obtained with good time at a high temperature, 10-15 minutes above 80°C, replenishing water constantly, and, additionally, putting in more reinforcing filler (it will not show a second peak in the DSC thermogram, see Figure 8). If the collagen is taken out before 70-75°C, the cycle was relatively short (less than 15-20 min). In the first grinding, the paste will be moderately ground (it will show a second peak in the DSC thermogram).

General characteristics and properties

General considerations:

Processability conditions, physicochemical characteristics and mechanical and viscoelastic properties of collagen and accordingly of products derived therefrom are determined fundamentally by three aspects:

1. Degree of collagen grinding: remaining crystalline clusters and final molecular weight.
2. Additive addition: amount and type of plasticizer and reinforcing fillers

3. Curing system: concentration and type of system.

The first aspect is fundamental. It conditions the final properties and processability characteristics. Like in most rubber and thermoplastic transformation processes, the melting temperature of the most heat-stable crystalline domains must be exceeded by at least 30-50°C. In completely denatured masses there are no crystalline domains above 50°C-70°C. This enables transformation by conventional techniques of compression, extrusion and injection molding. In these masses, the greater the degree of grinding the lower the molecular weight and the wider the molecular weight distribution. The mechanical properties will be worse, but subsequent processability improves. They tend to give stickier products. The difference in properties can be balanced out and corrected to a large extent by means of suitable additives and curing systems. It is mentioned in the literature that heat treatment at alkaline pH which hydrolyzes collagen and contributes to a wider molecular weight distribution (step 4); this is reduced if step 4 or heat treatment is avoided. However, the authors of the present invention have not observed variations in properties according to the variant used in preparing the skins (using or not using step 4, heat treatment) and have observed that the step which truly modifies the properties is the smaller or greater extent of grinding (step 8).

Stable crystalline structures remain in semi-ground masses. It is necessary to exceed the melting temperature of those crystalline domains, which entails higher transformation temperatures and the extrusion and injection molding are very limited: this complicates flow and there is a risk of degradation. Compression molding, however, is more feasible. In this case, high performance molded products (films) can be obtained sometimes without having to add additives to the collagen.

It can be said that:

- Semi-ground masses are more resistant to solvents (primarily water), harder, tougher and more rigid. Once cured, those parameters improve and the end product may swell just a little in water (less than 100%) and be insoluble. They need a subsequent step of adding more plasticizer for processing by injection and extrusion.
- Completely ground masses are easier to process but have lower hardness, rigidity and toughness values. They swell in water and can dissolve in a few hours. With a suitable curing system, they produce molded products that will swell more in water than preceding products but will be insoluble.
- In terms of fillers, both semi- or completely ground masses accept polar or semi-polar fillers very well (although mass that is ground more accepts such fillers better and in a larger amount): dry native collagen fibers, cellulose fibers, precipitated silica, etc. Besides the different types of curing systems conventionally used in the rubber industry: organic and inorganic peroxides, sulfur donors, accelerators, etc., it also accepts less polar fillers and plasticizers such as lubricating oils used in the leather industry.

It can also undergo subsequent tanning with conventional tanning systems. Therefore, by varying those three aspects mentioned above a very wide range of properties and characteristics of the obtained collagen can be achieved.

Examples of different systems with different properties are described below.

For the curable collagens of Examples 2 and 4, the curing agent is TMTD but it may well be zinc peroxide, $ZnO_2$ giving similar properties, although the curing temperature must be increased by 5 or 10°C.

If glycerin and another polar plasticizer are partially or completely replaced with lubricating oils such as those used in the leather industry, collagens which are more resistant to tear and abrasion, in addition to being more resistant to water, are formed.

The amounts seen in the examples are calculated for 1 kg of treated hide and are normally used in laboratory rollers. For industrial rollers, those amounts would be multiplied by 10 or by 20.

Properties derived from processing

Additive addition in the production process of the present invention is easier than in other conventional collagen transformation processes: addition of reinforcing fillers, cross-linking agents (curing agents), active ingredients and others.

High temperatures of more than 120°C can be reached during subsequent processing (extrusion, injection, compression, etc.), assuring sterilization.

The high crystallization capacity of collagen due to structural regularity (biopolymer) causes it to strengthen when stretched (comparable to natural rubber). Furthermore, its thermoplastic character makes extrusion into film and stretchable threads possible, which cannot be done with the conventional gel that maintains the native collagen structure.

Foaming is possible due to the water content: obtaining objects with an alveolar structure.

Due to the high water absorption capacity (without being cured), it can have a very low solid content, a gel being obtained.

The obtained paste gives rise to heat sealable films. If the grinding was intense, they are self-adhesive and stick to human skin.

It accepts inks very well due to the polar nature: it can be printed and written on.

Due to all these properties, the collagen of the invention has a primary application in the biomedical and cosmetic sector, although it can also be used in the food sector, in the paper industry, in the leather industry and in the packaging industry.

Properties of the material

The material is biodegradable, very similar to skin, edible and recyclable.

1. Biodegradable: depending on the additives, the material can last for days or years, it dissolves or only swells when immersed in water, being the first ever biodegradable collagen in existence.
2. Similar to skin: stretchable film which sticks to the skin can be made. It can be an active ingredient carrier and have healing power.
3. Edible: it can be transformed into a food casing.
4. Recyclable: the obtained parts can again be reprocessed, obtaining other geometric shapes.

EXAMPLES

Example 1: Thermoplastic collagen

(Figures 9 and 10)

Prior operations

1000 g of hide treated according to step 1: receiving the skins, step 2: washing the splits, step 3: swelling the skins, step 4: heat treatment and step 5: drying or wringing, are weighed.

The dry weight is calculated according to step 7 and calculations are made for obtaining collagen having a composition of 100 pbw collagen/25 pbw glycerin (plasticizer):

1000 g of hide → 330 g of collagen → 82.5 g of glycerin

It is appropriate to heat the rollers above 50°C.

Processing

I. The hide is passed several times through the rollers until it sticks to the rollers due to heat. In this step the rollers must be open so that the hide is not trapped and can pass through same. The objective of this step is to soften the hide by heating and for it to adhere to the rollers. It is necessary to prevent water from being wrung out and the temperature from dropping below 45°C. If the hide is not heated enough and/or is wrung excessively, band formation and accordingly subsequent grinding are impossible. In this case, native collagen fibers are released and a collagen fiber mass is formed (this is what should be done if collagen fibers are to be obtained).

II. The rollers move closer to one another little by little and the formation of an irregular pre-band and an irregular roll is seen. As the rollers gradually close in on each other, shearing forces increase and the band and the roll become more regular, i.e., they become increasingly similar to a continuous mass. The temperature as well as the solid content of the mass increase gradually.

III. When the band and the roll become even more consistent, 82.5 g of glycerin diluted in water up to half are added little by little. Denaturation and grinding continue. The collagen fibers decrease in size and start to no longer be visible. It is important to add glycerin when due to the internal friction of the material the temperature increases, because otherwise, when reducing the viscosity, the shearing forces decrease, the temperature drops and the process slows down excessively. When the hide is completely homogenous, the glycerin can be added at the end of step I, when most of the water remaining in the material evaporates (for the reasons described in section 8.3). In this case, the incorporation is much faster (2-5 minutes with respect to 10-15 minutes). If this is not the case, the glycerin is preferably added after step IV.

IV. When the band and the roll start to become shiny in the last stages of denaturation, the neutralizing agent is added: 50-60 ml of 2 N aqueous solution of $(NH_4)_2SO_4$. It is suitable to make cuts on one side of the band up to 2/3 (on the left) and pass it to the right and vice versa; and to remove it entirely and introduce it, rolled up, with the end facing down (end pass). This is repeated several times to achieve good homogenization after incorporating and dispersing the ingredients.

V. The paste is now a semi-ground paste. It can be removed and in a second step, grinding continues using cold rollers, as mentioned in sections 8.2 and 8.3. Otherwise, the method continues as described in section VI.

VI. The temperature is allowed to increase up to 75-80°C and is maintained for some time, 10-15 minutes, at this temperature, replenishing the water that is removed. Denaturation is now complete and prolonging it in time gives rise to a paste that has been increasingly ground with collagen having a lower molecular weight.

The band and the roll are consistent, white and shiny.

The cycle ends.

Composition and properties

The collagen has the following composition:

- 100 pbw collagen
- 25 pbw glycerin
- 5-10 pbw water (depending on the final grinding temperature)
- 3-8 pbw inorganic residue

and the following properties:

- It flows at temperatures between 80-120°C and a sticky film that readily adheres to the skin is obtained in a press. This film is not stretchable and swells in an aqueous solution and dissolves therein after a few hours.
- When it has cooled to room temperature, it has a hardness greater than 80 Shore A or 45 Shore D.
- It has a density of 1.20-1.24 g/cc.
- After a few hours it is in balance with ambient humidity, achieving 10-20 pbw of water, and hardness drops to below 60 Shore A.

The possible uses of the obtained collagen are:

a) Agricultural industry: biodegradable planting pots and packaging for seeds, among others.
b) Cosmetic industry: tampon casings, among others.
c) Pharmaceutical industry: hemostatic foams, dressings and patches with active ingredients, bandages, etc.
d) Paper industry: parchment paper for example.

Variant:

If in step III a solid reinforcing agent (ZnO, $CaSO_4$) at 2-3 pbw is added simultaneously with glycerin, grinding is faster and more effective. Once tempered for a few hours at room temperature:

the collagen then has the following composition:

- 100 pbw collagen
- 25 pbw glycerin
- 8-12 pbw inorganic residue
- 10-20 pbw water

and the following properties:

- It flows at temperatures between 80-120°C and a film which is no longer sticky is obtained in a press. This film is not stretchable and swells in an aqueous solution and takes longer to dissolve than in the preceding case.
- It has a hardness greater than 60 Shore A.
- It has a density of: 1.19-1.23 g/cc.
- Up to 40-50 pbw of water can be added in a subsequent step in cold conditions (temperatures not exceeding 55°C). When molded at 130-150°C, it gives rigid and soluble foams.
- With a greater amount of water and mechanical stirring it provides a soluble collagen gel.

The uses of the collagen obtained by this variant are similar to that obtained previously in this very example.

Example 2: Curable thermoplastic collagen

(Figures 11 and 12)

Prior operations

1000 g of hide treated according to steps 1, 2, 3, 4 and 5 are weighed.

The dry weight is calculated according to step 7 and calculations are made for obtaining collagen having a composition of 100 pbw collagen/33 pbw glycerin/5 pbw TMTD/4 pbw ZnO composition:

Example:

1000 g of hide → 330 g of collagen → 109 g of glycerin → 16.5 g of TMTD → 13.2 g of ZnO

It is appropriate to heat the rollers above 50°C.

Processing

Steps I, II and III are the same as the preceding example although in the third step glycerin and ZnO can be alternately added.

In the fourth step, neutralization is performed with the same amount of ammonium sulfate.

TMTD is added immediately after.

The cycle ends when the band and the roll are shiny and consistent.

Composition and properties

The collagen obtained in this mixture has the following composition

- 100 pbw collagen
- 33 pbw glycerin
- 5 pbw TMTD
- 4 pbw ZnO
- 5-10 pbw water which will become 10-20 pbw after a few hours at room temperature.

and the following properties:

- It flows at temperatures between 100-120°C and cures between 2-5 minutes at 120°C.
- This cured film is even less stretchable and swells in an aqueous solution but does not dissolve therein (it can swell less than 200%). These properties can be improved with post-curing at 60-80°C for a few hours or one day.
- When conditioned at room temperature the hardness is greater than 80 Shore A or 45 Shore D.
- Density: 1.24-1.28 g/cc.
- Like in Example 1, the collagen can be taken to levels of water of up to 50 pbw in a subsequent step in cold conditions, which allows obtaining rigid insoluble foam.

All these properties also depend on the concentration and on the type of curing system (in this case ZnO and TMTD). Higher levels of curing system entail greater hardness, lower film stretchability and less swelling.

The collagen obtained in this example can be used in the:

- Toy industry: dolls, balls.
- Paper industry: parchment paper.
- Leather industry: three-dimensional objects or objects having complex shape, binder for leather regeneration.
- Chewing gum base.

Example 3: Soluble elastomeric collagen

(Figures 13 and 14)

Prior Operations

1000 g of hide treated according to steps 1, 2, 3, 4 and 5 are weighed.

The dry weight is calculated according to step 7 and the calculations are made for obtaining collagen having a composition of 100 pbw collagen/66 pbw glycerin/20 pbw $CaSO_4$.

Example:

1000 g of hide → 330 g of collagen → 220 g of glycerin
→ 65 g of $CaSO_4$

It is appropriate to heat the rollers above 50°C.

Processing:

Steps I, II and III are the same as the preceding example although in the third step glycerin and calcium sulfate can be alternately added (or a suspension containing both glycerin and calcium sulfate can be added).

Neutralization is performed with the same amount of $(NH_4)_2SO_4$ solution.

It is ground like in the preceding cases.

The cycle ends when the band and the roll are shiny and consistent.

Composition and properties

The collagen obtained in this mixture has the following composition:

- 100 pbw collagen
- 66 pbw glycerin
- 20 pbw $CaSO_4$
- 40-60 pbw water (once balanced and conditioned at room temperature) and the following properties
- It flows at temperatures between 80-90°C
- A very stretchable film is obtained and it quickly swells in an aqueous solution and dissolves therein.
- It has a hardness between 25-35 Shore A.
- Density: 1.12-1.16 g/cc.
- It is susceptible to foaming, producing soft and soluble foam.

The obtained collagen can be used in the packaging sector: foams. In the field of medicine: ear plugs, membranes and others. In the pharmaceutical industry: for obtaining soft hemostatic foams, soft dressings and patches with active ingredients, and the like.

Example 4: Curable elastomeric collagen (gum)

(Figures 15 and 16)

Prior operations

1000 g of hide treated according to steps 1, 2, 3, 4 and 5 are weighed.

The dry weight is calculated according to step 7 and the calculations are made for obtaining collagen having a composition of 100 pbw collagen/66 pbw glycerin/20 pbw $CaSO_4$/5 pbw TMTD/4 pbw ZnO:

Example:

1000 g of hide → 330 g of collagen → 220 g of glycerin → 67 g of CaSO4
→ 16.5 g of TMTD → 13.2 g of ZnO

It is appropriate to heat the rollers above 50°C.

Processing

Steps I, II and III are the same as the preceding example although in the third step glycerin, the reinforcing filler and the curing system can be alternately added. It is best to add the glycerin, sulfate and zinc oxide in a suspension containing all three of them.

Like in the preceding examples, neutralization is performed in the fourth step and TMTD is added immediately after.

The cycle ends when the band and the roll are shiny and consistent.

Composition and properties

Composition:

- 100 pbw collagen
- 66 pbw glycerin
- 20 pbw $CaSO_4$
- 5 pbw TMTD
- 4 pbw ZnO
- 40-60 pbw water (once balanced and conditioned at room temperature)

Properties:

- It flows at temperatures between 80-90°C and cures between 2-5 minutes at 120°C. This cured film is stretchable and quickly recovers its shape when the force for stretching it is no longer applied (gum). It swells quickly in an aqueous solution but does not dissolve therein (it swells more than 400%). The properties improve with post-curing at 60-80°C for a few hours or one day.
- The hardness of the collagen is 30-40 Shore A
- Density: 1.15-1.19 g/cc
- It provides soft and insoluble foams.

All these properties largely depend on the concentration and on the type of curing system (in this case ZnO and TMTD). Higher levels of curing system entail greater hardness, lower film stretchability and less swelling.

The obtained collagen is suitable for use in the toy industry: in dolls, balls, masks and other toys of the same type.

Example 5: Semi-ground collagen

(Figures 17 and 18)

Prior operations:

100 g of hide treated according to steps 1, 2, 3, 4 and 5 are weighed.

The dry weight is calculated according to step 7 and the calculations are made for obtaining collagen having a composition of 100 pbw collagen/35 pbw glycerin.

Example:

100 g of hide → 33 g of collagen → 11.6 g of glycerin

Processing

Steps I, II and III are the same as the preceding examples.

Reinforcing filler is optionally added.

The paste is neutralized with 5-6 ml of ammonium sulfate and grinding continues for a while. The properties of the obtained collagen depend on this grinding time: the less the paste has been ground, the harder, more rigid and rougher it will be. They are stable in storage but will need a subsequent step of adding more plasticizer for processing.

The collagen is yellowish white in color and the band and the roll are somewhat less shiny, smooth and consistent than in the preceding examples.

It has a hardness of 70-80 Shore A or more, depending on the degree of grinding and a density of 1.13-1.17 g/cc.

The additives suitable for obtaining the systems and properties described in Examples 1, 2, 3 and 4 above will be added in the subsequent grinding step.

In this subsequent step it is appropriate to cool the rollers to obtain effective grinding and to achieve good and correct incorporation, dispersion and homogenization of the additives to be incorporated (cutting and end passing).

Composition:

- 100 pbw collagen
- 35 pbw glycerin
- 4-8 pbw inorganic residue
- 10-20 pbw water (once balanced and conditioned at room temperature)

**Claims**

1.  A process for obtaining a thermoplastic collagen paste from isolated animal skins including:

    1) Receiving and pre-treating the skins to obtain splits,
    2) Washing the splits to remove toxic or unknown water soluble substances,
    3) Swelling the skins to weaken the skin collagen,
    4) optionally, subjecting the skins to heat treatment to shorten the swelling step,
    5) Drying or wringing the skins to obtain a semi-dry treated skin having a water content between 35% - 65% and a hardness of 40 - 70 Shore A
    6) optionally, shaving the skins for tearing off the weakest, innermost layer of the skins by means of a shaving machine and
    7) Calculating the dry weight to calculate the amount of additives to be incorporated in the subsequent step,

    **characterized in that** the said process comprises a further subsequent step 8) comprising:subjecting the semi-dry treated skin of step 5) to a mechanical grinding treatment step in mixing cylinders in the presence of a plasticizer and a neutralizing agent at a temperature of 50 - 70°C for controlled denaturation and molecular weight reduction of the collagen present in the skin.

2.  The process for obtaining a thermoplastic collagen paste according to claim 1, **characterized in that** additional additives are incorporated in step (8) selected from the group consisting of curing agents, cross-linking agents, accelerators and/or activators, internal or external lubricants, foaming agents and reinforcing fillers.

3.  The process for obtaining a thermoplastic collagen paste according to claims 1-2, **characterized in that** the plasticizer of step (8) is glycerin, ethylene glycol and/or lubricating oils.

4.  The process for obtaining a thermoplastic collagen paste according to claims 1-3, **characterized in that** the neutralizing agent of step (8) is ammonium sulfate or ammonium chloride which is a solid or in a 2 N aqueous solution.

5.  The process for obtaining a thermoplastic collagen paste according to claims 1-4, **characterized in** the splits (2) are washed with a 2 g/l calcium hydroxide solution.

6.  The process for obtaining a thermoplastic collagen paste according to claims 1-5, **characterized in that** step (3) consisting of swelling the skins lasts from 3 to 8 days.

7. The process for obtaining a thermoplastic collagen paste according to claims 1-6, **characterized in that** step (3) consisting of swelling the skins lasts from 3 to 6 days at 20°C and at a drum rotational speed of 4-10 rpm at intermittent periods of stopping for one hour and rotating for one minute.

8. The process for obtaining a thermoplastic collagen paste according to claims 1-7, **characterized in that** the heat treatment process is carried out at a temperature of 45-55°C by rotating the drum at 2-3 rpm.

9. The process for obtaining a thermoplastic collagen paste according to claims 1-8, **characterized in that** drying was carried out between 12 hours and 5 days until the hide has a mean hardness between 40-70 Shore A.

10. The process for obtaining a thermoplastic collagen paste according to claims 1-9, **characterized in that** drying was carried out between 12 hours and 5 days until the hide has a mean hardness of the hide between 40-45 Shore A.

11. The process for obtaining a thermoplastic collagen paste according to claims 1-10, **characterized in that** shaving (6) is applied when the skins have a hardness between 30-60 Shore A and it consists of tearing off by means of a shaving machine the innermost layer of the skins, with which layer the process continues, the total process time thus being reduced to 2/3.

12. A thermoplastic collagen paste suitable for press molding, extrusion molding and injection molding obtainable by the process of any of claims 1-11, **characterized in that** it is in the form of a soft and stretchable gum, is biodegradable and recyclable and comprises denatured collagen, a plasticizer agent a neutralizing agent and optionally, additional additives selected from the group consisting of curing agents, cross-linking agents, accelerators and/or activators, internal or external lubricants, foaming agents and reinforcing fillers.

13. Use of the thermoplastic collagen paste according to claim 12 in the agricultural industry.

14. Use of the thermoplastic collagen paste according to claim 12 in the cosmetic industry.

15. Use of the thermoplastic collagen paste according to claim 12 in the pharmaceutical industry.

16. Use of the thermoplastic collagen paste according to claim 12 in the paper industry.

17. Use of the thermoplastic collagen paste according to claim 12 in the toy industry.

18. Use of the thermoplastic collagen paste according to claim 12 in the leather industry.

**Patentansprüche**

1. Verfahren zum Gewinnen einer thermoplastischen Kollagenmasse aus isolierten Tierhäuten, umfassend:

   1) Erhalten und Vorbehandeln der Häute zum Gewinnen von Spalten,
   2) Waschen der Spalten zum Entfernen von toxischen oder unbekannten wasserlöslichen Substanzen,
   3) Quellen der Häute zum Schwächen des Kollagens der Haut,
   4) wahlweise Unterziehen der Häute einer Wärmebehandlung, um den Quellschritt zu verkürzen,
   5) Trocknen oder Abwelken der Häute zum Gewinnen einer halbtrockenen, behandelten Haut, welche einen Wassergehalt von zwischen 35 % und 65 % und einer Härte von zwischen 40 und 70 Shore A aufweist
   6) wahlweise Falzen der Häute zum Abreißen der schwächsten, innersten Schicht der Häute mittels einer Falzmaschine und
   7) Berechnen des Trockengewichts zum Berechnen der Menge an Zusatzstoffen, welche in dem folgenden Schritt zuzugeben werden,

   **dadurch gekennzeichnet, dass** das Verfahren einen weiteren, folgenden Schritt (8) umfasst, der Schritt umfassend: Unterziehen der halbtrockenen, behandelten Haut aus Schritt (5) einem mechanischen Mahlbehandlungsschritt in Mischzylindem in Gegenwart eines Weichmachers und eines Neutralisationsmittels bei einer Temperatur von 50 bis 70 °C zur gesteuerten Denaturierung und Verringerung des Molekulargewichts des in der Haut anwesenden Kollagens.

2. Verfahren zum Gewinnen einer thermoplastischen Kollagenmasse nach Anspruch 1, **dadurch gekennzeichnet, dass** in Schritt (8) zusätzliche Zusatzstoffe, welche aus der Gruppe, bestehend aus Härtern, Vernetzern, Beschleunigern und/oder Aktivatoren, inneren oder äußeren Schmiermitteln, Schaumbildnern und Verstärkerfüllstoffen, ausgewählt sind, enthalten sind.

3. Verfahren zum Gewinnen einer thermoplastischen Kollagenmasse nach Anspruch 1 bis 2, **dadurch gekennzeichnet, dass** der Weichmacher aus Schritt (8) Glycerin, Ethylenglykol und/oder Schmieröle ist.

4. Verfahren zum Gewinnen einer thermoplastischen Kollagenmasse nach Anspruch 1 bis 3, **dadurch gekennzeichnet, dass** das Neutralisationsmittel aus Schritt (8) Ammoniumsulfat oder Ammoniumchlorid ist, welches ein Feststoff ist oder sich in einer 2 N wässrigen Lösung befindet.

5. Verfahren zum Gewinnen einer thermoplastischen Kollagenmasse nach Anspruch 1 bis 4, **dadurch gekennzeichnet, dass** die Spalten (2) mit einer 2 g/l Calciumhydroxid-Lösung gewaschen werden.

6. Verfahren zum Gewinnen einer thermoplastischen Kollagenmasse nach Anspruch 1 bis 5, **dadurch gekennzeichnet, dass** Schritt (3), bestehend aus dem Quellen der Häute, 3 bis 8 Tage dauert.

7. Verfahren zum Gewinnen einer thermoplastischen Kollagenmasse nach Anspruch 1 bis 6, **dadurch gekennzeichnet, dass** Schritt (3), bestehend aus dem Quellen der Häute, 3 bis 6 Tage bei 20 °C und einer Trommeldrehzahl von 4 bis 10 rpm mit intermittierenden Phasen des Stoppens für eine Stunde und des Drehens für eine Minute dauert.

8. Verfahren zum Gewinnen einer thermoplastischen Kollagenmasse nach Anspruch 1 bis 7, **dadurch gekennzeichnet, dass** das Verfahren der Wärmebehandlung bei einer Temperatur von 45 bis 55 °C unter Drehen der Trommel bei 2 bis 3 rpm ausgeführt wird.

9. Verfahren zum Gewinnen einer thermoplastischen Kollagenmasse nach Anspruch 1 bis 8, **dadurch gekennzeichnet, dass** das Trocknen zwischen 12 Stunden und 5 Tagen, bis das Leder eine Durchschnittshärte von zwischen 40 und 70 Shore A aufweist, ausgeführt wird.

10. Verfahren zum Gewinnen einer thermoplastischen Kollagenmasse nach Anspruch 1 bis 9, **dadurch gekennzeichnet, dass** das Trocknen zwischen 12 Stunden und 5 Tagen, bis das Leder eine Durchschnittshärte des Leders zwischen 40 und 45 Shore A aufweist, ausgeführt wird.

11. Verfahren zum Gewinnen einer thermoplastischen Kollagenmasse nach Anspruch 1 bis 10, **dadurch gekennzeichnet, dass** das Falzen (6) angewendet wird, wenn die Häute eine Härte zwischen 30 und 60 Shore A aufweisen, und es aus dem Abreißen der innersten Schicht der Häute mittels einer Falzmaschine besteht, mit welcher Schicht das Verfahren fortgesetzt wird, so dass die Gesamtzeit des Verfahrens auf 2/3 verringert wird.

12. Eine thermoplastische, zum Pressformen, Extrudieren und Spritzgießen geeignete Kollagenmasse, welche durch das Verfahren von einem der Ansprüche 1 bis 11 gewinnbar ist, **dadurch gekennzeichnet, dass** sie in der Form eines weichen und dehnbaren Gummis ist, biologisch abbaubar und wiederverwertbar ist und denaturiertes Kollagen, ein Weichmachermittel und ein Neutralisationsmittel und wahlweise zusätzliche Zusatzstoffe, welche aus der Gruppe, bestehend aus Härtern, Vernetzern, Beschleunigern und/oder Aktivatoren, inneren oder äußeren Schmiermitteln, Schaumbildnern und Verstärkerfüllstoffen, ausgewählt sind, umfasst.

13. Verwendung einer thermoplastischen Kollagenmasse nach Anspruch 12 in der Agrarindustrie.

14. Verwendung einer thermoplastischen Kollagenmasse nach Anspruch 12 in der Kosmetikindustrie.

15. Verwendung einer thermoplastischen Kollagenmasse nach Anspruch 12 in der Pharmaindustrie.

16. Verwendung einer thermoplastischen Kollagenmasse nach Anspruch 12 in der Papierindustrie.

17. Verwendung von einer thermoplastischen Kollagenmasse nach Anspruch 12 in der Spielzeugindustrie.

**18.** Verwendung von einer thermoplastischen Kollagenmasse nach Anspruch 12 in der Lederindustrie.

**Revendications**

**1.** Procédé pour obtenir une pâte de collagène thermoplastique à partir de peaux d'animaux isolées comportant :

> 1) La réception et le prétraitement des peaux pour obtenir des croûtes,
> 2) Le lavage des croûtes pour éliminer les substances solubles dans l'eau toxiques ou inconnues,
> 3) Le gonflement des peaux pour affaiblir le collagène de la peau,
> 4) optionnellement, la soumission des peaux à un traitement thermique pour raccourcir l'étape de gonflement,
> 5) Le séchage ou l'essorage des peaux pour obtenir une peau traitée demi-sèche ayant une teneur en eau entre 35 % - 65 % et une dureté de 40 - 70 Shore A
> 6) optionnellement, le rasage des peaux pour arracher la couche la plus fragile, la plus interne des peaux au moyen d'une machine à raser et
> 7) Le calcul du poids sec pour calculer la quantité d'additifs à incorporer à l'étape ultérieure,

> **caractérisé en ce que** ledit procédé comprend une étape ultérieure supplémentaire 8) comprenant : la soumission de la peau traitée demi-sèche de l'étape 5) à une étape de traitement de meulage mécanique dans des cylindres de malaxage en présence d'un plastifiant et d'un agent neutralisant à une température de 50 - 70 °C pour la dénaturalisation contrôlée et la réduction du poids moléculaire du collagène présent dans la peau.

**2.** Procédé pour obtenir une pâte de collagène thermoplastique selon la revendication 1, **caractérisé en ce que** des additifs additionnels sont incorporés à l'étape (8) choisis dans le groupe consistant en des agents de durcissement, des agents de réticulation, des accélérateurs et/ou des activateurs, des lubrifiants internes ou externes, des agents moussants et des charges de renforcement.

**3.** Procédé pour obtenir une pâte de collagène thermoplastique selon les revendications 1-2, **caractérisé en ce que** le plastifiant de l'étape (8) est la glycérine, le glycol d'éthylène et/ou les huiles lubrifiants.

**4.** Procédé pour obtenir une pâte de collagène thermoplastique selon les revendications 1-3, **caractérisé en ce que** l'agent neutralisant de l'étape (8) est le sulfate d'ammonium ou le chlorure d'ammonium qui est un solide ou dans une solution aqueuse 2 N.

**5.** Procédé pour obtenir une pâte de collagène thermoplastique selon les revendications 1-4, **caractérisé en ce que** les croûtes (2) sont lavées avec une solution d'hydroxyde de calcium de 2 g/l.

**6.** Procédé pour obtenir une pâte de collagène thermoplastique selon les revendications 1-5, **caractérisé en ce que** l'étape (3) consistant à faire gonfler les peaux dure de 3 à 8 jours.

**7.** Procédé pour obtenir une pâte de collagène thermoplastique selon les revendications 1-6, **caractérisé en ce que** l'étape (3) consistant à faire gonfler les peaux dure de 3 à 6 jours à 20 °C et à une vitesse de rotation de tambour de 4-10 tr/min à des périodes intermittentes d'arrêt pendant une heure et de rotation pendant une minute.

**8.** Procédé pour obtenir une pâte de collagène thermoplastique selon les revendications 1-7, **caractérisé en ce que** le procédé de traitement thermique est réalisé à une température de 45-55 °C en faisant tourner le tambour à 2-3 tr/min.

**9.** Procédé pour obtenir une pâte de collagène thermoplastique selon les revendications 1-8, **caractérisé en ce que** le séchage a été réalisé entre 12 heures et 5 jours jusqu'à ce que le cuir ait une dureté moyenne entre 40-70 Shore A.

**10.** Procédé pour obtenir une pâte de collagène thermoplastique selon les revendications 1-9, **caractérisé en ce que** le séchage a été réalisé entre 12 heures et 5 jours jusqu'à ce que le cuir ait une dureté moyenne du cuir entre 40-45 Shore A.

**11.** Procédé pour obtenir une pâte de collagène thermoplastique selon les revendications 1-10, **caractérisé en ce que** le rasage (6) est appliqué lorsque les peaux ont une dureté entre 30-60 Shore A et il consiste à arracher au moyen d'une machine à raser la couche la plus interne des peaux, couche avec laquelle le procédé continue, la durée du procédé totale étant donc réduite au 2/3.

12. Pâte de collagène thermoplastique apte au moulage à la presse, au moulage par extrusion et au moulage par injection pouvant être obtenue par le procédé selon l'une des revendications 1-11, **caractérisée en ce qu'**elle est sous la forme d'une gomme molle et extensible, elle est biodégradable et recyclable et elle comprend un collagène dénaturé, un agent plastifiant, un agent neutralisant et optionnellement, des additifs additionnels choisis dans le groupe consistant en des agents de durcissement, des agents de réticulation, des accélérateurs et/ou des activateurs, des lubrifiants internes ou externes, des agents moussants et des charges de renforcement.

13. Utilisation de la pâte de collagène thermoplastique selon la revendication 12 dans l'industrie de l'agriculture.

14. Utilisation de la pâte de collagène thermoplastique selon la revendication 12 dans l'industrie du cosmétique.

15. Utilisation de la pâte de collagène thermoplastique selon la revendication 12 dans l'industrie pharmaceutique.

16. Utilisation de la pâte de collagène thermoplastique selon la revendication 12 dans l'industrie papetière.

17. Utilisation de la pâte de collagène thermoplastique selon la revendication 12 dans l'industrie du jouet.

18. Utilisation de la pâte de collagène thermoplastique selon la revendication 12 dans l'industrie de la fourrure.

**DSC**

69.97°C

79.57°C

94.68°C

64.08°C
21.97(33.15)J/g
38.77 J/g col

11.18J/g
19.72 J/g col

Heat flow (w/g)

Temperature (°C)

# FIG. 1

DSC

FIG. 2

**FIG. 3**

FIG. 4

FIG. 5

DSC

FIG. 6

**FIG. 7**

DSC

2nd melting peak
does not flow at 80°C

no 2nd melting peak
flows at 80°C

Heat flow (w/g)

0.25  0.20  0.15  0.10  0.05

Temperature (°C)

0  20  40  60  80  100  120  140

FIG. 8

**DSC**

**FIG. 9**

EP 2 727 938 B1

FIG. 10

EP 2 727 938 B1

**DSC**

**FIG. 11**

FIG. 12

**DSC**

**FIG. 13**

FIG. 14

FIG. 15

FIG. 18

FIG. 17

**FIG. 18**

EP 2 727 938 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4185011 A
- US 4404033 A
- ES 457823
- US 3652381 A
- EP 1852529 A1
- ES 496945
- ES 2340606 T3

- EP 0256663 A
- EP 1270672 A
- US 2011020864 A
- GB 2078797 A
- WO 2011022794 A
- WO 2007104322 A